# EUROPÄISCHE PATENTSCHRIFT

(11) **EP 2 124 786 B1**
(45) Veröffentlichungstag und Bekanntmachung des Hinweises auf die Patenterteilung: **03.12.2014**
(21) Anmeldenummer: 07856569.4
(22) Anmeldetag: 11.12.2007
(51) Int. Cl.: A61B 17/80, A61B 17/00

(54) **KRAFTTRÄGER FÜR EIN KNOCHENFIXATIONSSYSTEM**
LOAD CARRIER FOR A BONE FIXATION SYSTEM
PLAQUE SUPPORT POUR SYSTÈME DE FIXATION OSSEUSE

(30) Priorität: 20.12.2006 DE 102006060935
(43) Veröffentlichungstag der Anmeldung: 02.12.2009
(73) Patentinhaber: Wolter, Dietmar, 22955 Hoisdorf (DE)
(72) Erfinder: Wolter, Dietmar, 22955 Hoisdorf (DE)
(74) Vertreter: Glawe, Delfs, Moll
(86) Internationale Anmeldenummer: PCT/EP2007/010821
(87) Internationale Veröffentlichungsnummer: WO 2008/077482

(56) Entgegenhaltungen:
- EP-A- 0 266 146
- EP-A1- 0 448 233
- WO-A-01/19264
- DE-A1- 2 806 414
- DE-A1- 4 343 117
- US-A- 3 463 148
- US-A- 4 454 876
- HORN, H.: 'Werkstoffkunde Teil 5' INTERNET CITATION, [Online] 31 Mai 2005, Gefunden im Internet: <URL:http://www.haw-hamburg.de/fileadmin/us er_upload/IWS/PDF/Skript_teil05.pdf> [gefunden am 2012-02-08]

## Beschreibung

Die Erfindung betrifft einen Kraftträger für ein Knochenfixationssystem und ein Verfahren zu seiner Herstellung.

Fixationssysteme für Knochen mit einem Kraftträger und in den Kraftträger einsetzbaren und fixierbaren Knochenschrauben, beispielsweise Platten-, Nagel- und Fixateur-Systeme, werden verwendet um Knochenbruchstücke operativ miteinander zu verbinden.

Knochenschraube und Knochenplatte können dabei auch winkelstabil miteinander verblockt sein. Diese neue Generation von Implantaten, mit einer multidirektional winkelstabilen Verbindung zwischen dem Schraubenkopf und dem Knochenplattenloch, zeichnen sich durch eine höhere Leistungsfähigkeit bei gleichem oder geringerem Materialeinsatz aus.

Untersuchungen haben gezeigt, dass der Kraftfluss in diesen winkelstabilen Systemen dadurch gekennzeichnet ist, dass die Kräfte nicht gleichmäßig von einem Knochenfragment über die Schrauben in die Knochenplatte und über die Platte und Schrauben auf das andere Knochenfragment übertragen werden, sondern dass die erste dem Bruch benachbarte Schraube und die das Plattenloch umgebenden Plattenanteile ca. 50-60 % der Gesamtkräfte und -lasten, die folgende, also zweite Schraube und die das Plattenloch umgebenden Plattenanteile 20-30 % und die nächste, also dritte Schraube und die das Plattenloch umgebenden Plattenanteile ca. 10 % übertragen.

Diese Tatsache ist deshalb von besonderer Bedeutung, weil bei Übernahme des bisher genutzten Plattendesigns ein Versagen des Systems im Bereich des ersten dem Bruch benachbarten Plattenloches droht und zwar in der Weise, dass entweder die Schraube im Halsbereich oder die Platte auf Höhe dieses ersten Loches sich verformt bzw. durch Überlastung oder Ermüdung bricht.

Im europäischen Patent 1 211 994 B1 wird diese Problematik in der Weise gelöst, dass der Kraftträger eine Verstärkung in Form einer Verbreiterung oder einen Bereich mit festerem Werkstoff aufweist. Am stärksten ist dieses im Bereich des ersten, proximal an der Bruch- oder Instabilitätszone gelegenen Loches für die Knochenschraube, geringer im Bereich des zweiten, distal nachfolgenden Loches und am geringsten im Bereich des dritten, distal nachfolgenden Loches.

Weiterhin kann durch eine gleichzeitige Verdickung des Schraubenkernes und damit der Schraube reagiert werden, so dass die Belastungsfestigkeit der jeweiligen Plattenloch-Schrauben-Einheit eine Addition der Festigkeit der Platte und der Festigkeit der Schraube darstellt.

Der Nachteil liegt in einer größeren Dimensionierung und damit in einer stärkeren Abdeckung der Knochenoberfläche und in einem stärkeren Abheben der Weichteile, die über der Platte zu liegen kommen. Dieses ist gleichbedeutend mit einer Vergrößerung der OP-Wunde und somit auch mit einem größeren OP-Aufwand. Für bestimmte Einsatzgebiete, bei denen der Größe der Implantate anatomische Grenzen gesetzt sind, z.B. im Handbereich, sind die in der EP 1 211 994 B1 offenbarten Fixationssysteme, nicht zuletzt wegen der zusätzlichen Verbreiterung bzw. Verdickung des Kraftträgers, kaum einsetzbar.

EP 044 8233 zeigt einen Kraftträger gemäß des Oberbegriffs von Anspruch 1. DE 4343117 zeigt die Verblockung von Schraubenkopf und hoch.

Der Erfindung liegt die Aufgabe zugrunde, einen Kraftträger für ein Knochenfixationssystem zu schaffen, der die vorgenannten Nachteile weitestgehend vermeidet.

Die Erfindung löst diese Aufgabe mit einem Kraftträger mit den Merkmalen des Anspruchs 1 und einem Verfahren mit den Merkmalen des Anspruchs 6. Vorteilhafte Ausführungsformen der Erfindung sind in den Unteransprüchen offenbart.

Die Erfindung hat erkannt, dass ein einstückiger Kraftträger mit lokal unterschiedlichen Materialfestigkeiten in verschiedenen Bereichen des Kraftträgers bei gleich bleibender Materialelastizität im Hinblick auf den Kraftfluss am Implantat angepasst werden kann. Wird die Materialfestigkeit des Kraftträgers der jeweiligen an den verschiedenen Bereichen des Kraftträgers anliegenden Belastung der Bereiche angepasst, kann man auf die aus der EP 1 211 994 B1 bekannten Verbreiterungen und Verdickungen des Kraftträgerquerschnitts weitestgehend verzichten und die dadurch kleiner dimensionierten und dennoch verstärkten Kraftträger auch in kritischen Bereichen, beispielsweise für Handimplantate, verwenden.

Die Erfindung hat des Weiteren erkannt, dass sich für den Kraftträger Titan eignet. Dieses Material lässt sich besonders gut in seinen Materialeigenschaften durch Materialverdichtung so verändern, dass daraus eine dem Kraft- und Lastfluss korrespondierende Festigkeitserhöhung des Kraftträgers bei gleich bleibender Elastizität resultiert. Die gleich bleibende Materialelastizität, d.h. die Fähigkeit einer einwirkenden Kraft einen mechanischen Widerstand entgegen zu setzen und nach dem Entlasten wieder seine Ausgangsform einzunehmen, ist ein wesentliches Merkmal für den Kraftträger. Durch die gleich bleibende Elastizität, selbst in Bereichen mit erhöhter Materialfestigkeit, weist der erfindungsgemäße Kraftträger keine Bereiche mit erhöhter Materialspröde auf, die bei Belastung als erstes mit einem Versagen (Bruch) reagieren würden.

Der erfindungsgemäße Kraftträger, der sich einerseits durch lokal unterschiedliche Materialfestigkeiten und andererseits durch eine gleich bleibende Materialelastizität auszeichnet, lässt sich durch Verformung eines Kraftträgerhalbzeugs, welches in den Bereichen mit einer vorgesehenen hohen Materialfestigkeit des Kraftträgers ein größeres Materialvolumen aufweist, als in den Bereichen mit einer vorgesehenen geringeren Materialfestigkeit in einem formgebenden Pressformwerkzeug, herstellen. Das Pressformwerkzeug weist ein Oberwerkzeug und ein Unterwerkzeug auf, zwischen denen das Kraftträgerhalbzeug verformt wird. Die mit dem Kraftträger beim Verformen in Kontakt tretenden Oberflächen des Oberwerkzeugs und des Unterwerkzeugs können plane Oberflächen oder Ein- oder Ausformungen aufweisen, die sich nach der Verformung in dem Pressformwerkzeug spiegelsymmetrisch in der Oberfläche des Kraftträgers, vorzugsweise in den Bereichen mit einer vorgesehenen hohen Materialfestigkeit, wiederfinden.

Zweckmäßigerweise ist die Verformung eine Kaltumformung, d.h. bei Metallwerkstoffen eine formgebende Umformung bei einer Temperatur deutlich unterhalb der Rekristallisationstemperatur, beispielsweise bei Raumtemperatur. Die dabei auf das Halbzeug in dem Pressformwerkzeug einwirkende Umformkraft verringert die Höhe und/oder Breite bzw. des Querschnitts des Halbzeugs in den Bereichen mit einer vorgesehenen hohen Materialfestigkeit. Dabei kann auf das Kraftträgerhalbzeug ein einachsiger Druck, wie er bei Stauchen auftritt, oder auch ein zweiachsiger Druck, wie er beim Walzen auftritt, aufgebracht werden. Letzteres hat bei der Verwendung eines titanhaltigen Werkstoffs den Vorteil, dass sich neben der Verdichtung des Werkstoffs beim Walzen die Fasern in Walzrichtung ausrichten, was einen zusätzlichen Stabilitätsgewinn ermöglicht, da die Fasern durch eine vorgegebene Walzrichtung gezielt in Richtung der Hauptbelastungsachse des Kraftträgers ausgerichtet werden können.

Der erfindungsgemäße Kraftträger weist mindestens zwei benachbarte Löcher zur Aufnahme von Knochenschrauben auf. Die Löcher sind vorzugsweise mit einem für eine Verblockung mit einer Knochenschraube geeignetem Mittel, vorzugsweise einer Materiallippe, versehen. Dies lässt sich in dem erfindungsgemäßen Verfahren zur Herstellung eines solchen Kraftträgers bereits bei der Verformung des Kraftträgerhalbzeugs berücksichtigen, indem das Oberwerkzeug und/oder Unterwerkzeug Aufnahmen, vorzugsweise Bohrungen, aufweisen, die eine wesentliche Materialverfestigung in den Bereichen des Kraftträgerhalbzeugs verhindern, die den Aufnahmen bei der Verformung in Schritt b) gegenüberliegen und in denen einzubringende Löcher für die Befestigung des Kraftträgers an dem Knochen vorgesehen sind.

Durch die für die Knochenschrauben vorgesehen und weitestgehend nicht materialverfestigten Bereiche lassen sich die entsprechenden Löcher bohren und in dem Randbereich der Löcher mit einem Mittel für die Verblockung mit den Knochenschrauben, einer Materiallippe, versehen.

Wie eingangs erwähnt, sind neben dem die Bruch- oder Instabilitätszone überbrückenden Bereich des Kraftträgers insbesondere die Bereiche an den ersten Löchern des Kraftträgers an der Bruch- oder Instabilitätszone besonders anfällig für einen Bruch. Über diesen Bereich werden ca. 50-60% der Gesamtkräfte und - lasten übertragen. Gemäß einer bevorzugten Ausführungsform der Erfindung weist dieser besonders stark beanspruchte Bereich eine erhöhte Materialfestigkeit auf. Diese ist vorzugsweise in dem am stärksten belasteten Bereich um mindestens 40%, vorzugsweise um mindestens 60%, weiter vorzugsweise um mindestens 80% gegenüber der Materialfestigkeit in diesem Bereich vor der Verformung erhöht.

An gegebenenfalls weiteren distal von den ersten Löchern gelegenen Löchern braucht der Kraftträger keine Bereiche erhöhter Materialfestigkeit aufzuweisen. Da man jedoch davon ausgehen kann, dass wie eingangs erwähnt, der Kraftträger auch an den folgenden Löchern eine höhere Belastung aufnehmen muss, weist auch das nächstfolgende Loch und dass gegebenenfalls weitere nächstfolgende Loch in den Bereichen an den Löchern eine erhöhte Materialfestigkeit auf, die entsprechend der Belastung dieser Bereiche mit zunehmenden Abstand von der Bruch- oder Instabilitätszone des Knochens abnimmt. So ist beispielsweise bei einem Kraftträger die Materialfestigkeit in dem Bereich an dem zweiten Loch nur etwa halb so stark, wie die Materialfestigkeit in dem Bereich an dem ersten Loch und die Materialfestigkeit in dem Bereich an dem dritten Loch ist höchstens halb so stark, wie die Materialfestigkeit in dem Bereich an dem zweiten Loch. Zweckmäßigerweise ändert sich die Materialfestigkeit in dem Kraftträger bei dem Übergang von Bereichen erhöhter Materialfestigkeit in Bereiche geringerer Materialfestigkeit fließend.

Die Erfindung wird nachfolgend anhand der anliegenden Zeichnungen von Ausführungsbeispielen näher erläutert. Die Zeichnungen zeigen:
- Fig. 1: eine erfindungsgemäße 6-Loch-Knochenplatte in der Draufsicht;
- Fig. 2: ein 6-Loch-Knochenplattenhalbzeug im Längsschnitt;
- Fig. 3: ein 6-Loch-Knochenplattenhalbzeug im Längsschnitt nach der Verformung;
- Fig. 4: eine erfindungsgemäße 6-Loch-Knochenplatte im Längsschnitt;
- Fig. 5: eine gehärtete Pressform für eine 6-Loch-Knochenplatte, die auf ein Knochenplattenhalbzeug einwirkt;
- Fig. 6: einen Querschnittsausschnitt aus Figur 5 bei der Umformung;
- Fig. 7: einen Querschnittsausschnitt des Knochenplattenhalbzeugs nach der Umformung;
- Fig. 8: ein Querschnittsausschnitt durch ein Loch einer verfestigten 6-Loch-Knochenplatte.

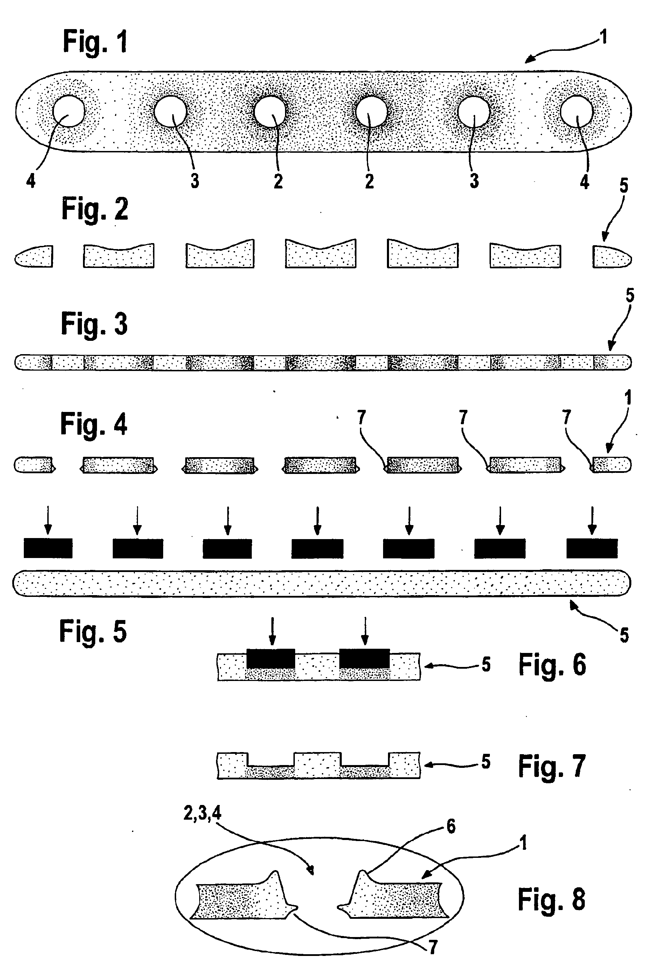

Figur 1 zeigt eine 6-Loch-Knochenplatte 1, wie sie aus dem 6-Loch Knochenplattenhalbzeug 5, wie in Figur 2 dargestellt, erhalten werden kann. Die Knochenplatte 1 weist jeweils ein Paar Löcher 2, 3 und 4 auf. Davon ist das Plattenloch 2 proximal, das Plattenloch 4 distal und das Plattenloch 3 zwischen den Plattenlöchern 2 und 4 in Bezug auf die Bruch- oder Instabilitätszone eines Knochens angeordnet. Um das Loch 2 hat die Knochenplatte 1 einen Bereich mit erhöhter Materialfestigkeit, um das Loch 3 einen Bereich geringerer Materialfestigkeit und um das Loch 4 einen Bereich noch geringerer Materialfestigkeit gegenüber dem Bereich um das Loch 2. Die Materialfestigkeit dieser Bereiche entspricht ihrer Lastübertragung, die in dem Bereich um das Plattenloch 2 ca. 50-60%, in dem Bereich um das Plattenloch 3 ca. 20-30% und in dem Bereich um das Plattenloch 4 ca. 10% beträgt. Die Stärke der Materialfestigkeit ist durch die entsprechende Stärke der Schattierung schematisch dargestellt. In den Bereichen mit starker Schattierung ist die Materialfestigkeit am stärksten. Die Knochenplatte 1 weist eine plane Ober- und Unterseite auf. Die unterschiedliche Materialfestigkeit der Bereiche der Knochenplatte 1 entspricht dem in diesen Bereichen bei dem Knochenplattenhalbzeug 5 vorliegendem Materialvolumen vor dem Pressvorgang zur Materialverdichtung, wie in Figur 2 dargestellt. Wie aus dem seitlichen Schnittbild des Knochenplattenhalbzeugs 5 in Figur 2 ersichtlich, weist das Knochenplattenhalbzeug 5 unterschiedliche Dicken und Löcher auf. Das Knochenplattenhalbzeug 5 kann durch ein Formgebendes Pressformwerkzeug mit planen,Flächen ober- und unterseitig plan geformt werden, so dass ein Teil des Materials bei der Verformung in die Löcher des Knochenplattenhalbzeugs 5 hineinfließt.

Das nach der Verformung erhaltene Knochenplattenhalbzeug 5 ist in Figur 3 dargestellt. Das in die Löcher bei der Verformung hinein geflossene Material ist nicht wesentlich verfestigt, so dass hieraus in einer Nachbearbeitung eine weiche Materiallippe 7 zum Umformen und Schraubenkopfverblocken herausgearbeitet werden kann, wie in Figur 4 dargestellt.

Figur 5 zeigt ein weiches Knochenplattenhalbzeug 5, auf das ein eine gehärtete Pressform mit Löchern in Pfeilrichtung aufgepresst wird. Bei dem Pressvorgang fließt das weiche Material des Knochenplattenhalbzeugs 5 in die Löcher des Formwerkzeugs zurück.

Figur 6 zeigt im Ausschnitt den Materialrückfluss in die Löcher beim Aufpressen der Pressform auf das weiche Knochenplattenhalbzeug 5 unter Bildung des in Figur 7 ersichtlichen Materialpilzes am umgeformten Knochenplattenhalbzeug 5.

Figur 8 zeigt den Bereich des Materialpilzes aus Figur 7, der nachbearbeitet wurde. Das weiche Material des Materialpilzes dient als Material der Lochwand der Knochenplatte 1, aus dem eine horizontale Materiallippe 7 und ein ringförmiger Wall 6 um das Loch 2, 3, 4 herausgearbeitet wurde. Der ringförmige Wall 6 erhöht die Festigkeit im Lochbereich und schirmt den Schraubenkopf (nicht dargestellt) gegenüber dem aufliegendem Gewebe (nicht dargestellt) ab, damit dieses nicht durch scharfe Teile des Schraubenkopfes irritiert wird.

## Patentansprüche

1. Kraftträger (1) für ein Knochenfixationssystem, wobei der Kraftträger (1) aus Titan besteht, wobei der Kraftträger (1) lokal unterschiedliche Materialfestigkeit bei gleich bleibender Materialelastizität aufweist, dass der Kraftträger (1) mindestens zwei benachbarte Löcher (2, 3, 4) zur Aufnahme von Knochenschrauben aufweist, dass das Material des Kraftträgers (1) im Bereich der Löcher (2, 3, 4) eine erhöhte Materialfestigkeit aufweist, **dadurch gekennzeichnet, dass** die Löcher (2, 3, 4) mit einem für eine Verblockung mit einer Knochenschraube geeigneten Mittel in Form einer nicht materialverfestigten Materiallippe (7) versehen sind.

2. Kraftträger (1) nach Anspruch 1, **dadurch gekennzeichnet, dass** die lokal unterschiedliche Materialfestigkeit der jeweiligen Belastung dieser Bereiche entspricht.

3. Kraftträger (1) nach Anspruch 1 oder 2 einem der vorangehenden Ansprüche, **dadurch gekennzeichnet, dass** der Kraftträger (1) zwei benachbarte Löcher (2) zur Aufnahme von Knochenschrauben aufweist, die auf verschiedenen Seiten einer Bruch- oder Instabilitätszone eines Knochens anzuordnen sind und der Kraftträger (1) in den Bereichen an den benachbarten Löcher (2) oder in den Bereichen an den benachbarten Löcher (2) und dem die Bruch- oder die Instabilitätszone überbrückenden Bereich zwischen den benachbarten Löchern (2) eine lokal erhöhte Materialfestigkeit aufweist.

4. Kraftträger (1) nach Anspruch 3, **dadurch gekennzeichnet, dass** der Kraftträger (1) mindestens ein weiteres Loch (3) aufweist, welches gegenüber den ersten benachbarten Löchern (2) distaler von der Bruch- oder Instabilitätszone des Knochens angeordnet ist und dass der Kraftträger (1) in dem Bereich an dem weiteren Loch (3) ebenfalls eine erhöhte Materialfestigkeit durch Materialverdichtung aufweist, diese jedoch geringer, vorzugsweise nur etwa halb so stark, wie die Materialfestigkeit im Bereich an den ersten benachbarten Löchern (2) ist.

5. Kraftträger (1) nach Anspruch 4, **dadurch gekennzeichnet, dass** der Kraftträger (1) mindestens ein weiteres Loch (4) aufweist, das gegenüber dem zweiten Loch (3) distaler von der Bruch- oder Instabilitätszone des Knochens angeordnet ist und vorzugsweise in dem Bereich an dem weiteren Loch (4) eine erhöhte Materialfestigkeit durch Materialverdichtung aufweist, diese jedoch geringer, vorzugsweise höchstens halb so stark, wie die Materialfestigkeit im Bereich an dem zweiten Loch (3) ist.

6. Verfahren zur Herstellung eines Kraftträgers (1) nach einem der Ansprüche 1 bis 5, mit den folgenden Schritten:
a) Bereitstellung eines Kraftträgerhalbzeugs (5) aus Titan, welches in den Bereichen mit einer vorgesehenen hohen Materialfestigkeit des Kraftträgers (1) ein größeres Materialvolumen aufweist, als in den Bereichen mit einer vorgesehenen geringeren Materialfestigkeit;
b) Verformung des Kraftträgerhalbzeugs (5) mittels eines formgebenden Pressformwerkzeugs, so dass das Kraftträgerhalbzeug (5) zwischen einem die Form des Kraftträgers vorgebenden Oberwerkzeug und Unterwerkzeug verformt wird und dass das Oberwerkzeug und/oder Unterwerkzeug Aufnahmen, vorzugsweise Bohrungen, aufweisen, die eine wesentliche Materialverfestigung in den Bereichen des Kraftträgerhalbzeugs (5) verhindern, die den Aufnahmen bei der Verformung in Schritt b) gegenüberliegen und in denen einzubringende Löcher (2, 3, 4) für die Befestigung des Kraftträgers (1) an dem Knochen vorgesehen sind;
c) Lochen der Bereiche, welche den Aufnahmen bei der Verformung in Schritt b) gegenüberliegen und in denen Löcher für die Befestigung des Kraftträgers (1) an dem Knochen vorgesehen sind;
d) Versehen der nicht materialverfestigten Lochwand mit einer Materiallippe.

7. Verfahren nach Anspruch 6, **dadurch gekennzeichnet, dass** die Verformung durch Pressen im Wege der Kaltverformung, vorzugsweise bei Raumtemperatur, erfolgt.

8. Verfahren nach Anspruch 6 oder 7, **dadurch gekennzeichnet, dass** in Schritt b) die Höhe des Kraftträgerhalbzeugs (5) in den Bereichen mit einer vorgesehenen hohen Materialfestigkeit senkrecht zur Umformkraft verringert wird.

9. Verfahren nach einem der Ansprüche 6 bis 8, **dadurch gekennzeichnet, dass** das Oberwerkzeug und/oder das Unterwerkzeug Ein- oder Ausformungen, vorzugsweise in den korrespondierenden Bereichen des Kraftträger (1) mit einer vorgesehenen hohen Materialfestigkeit, aufweisen, die sich spiegelsymmetrisch in der Oberfläche des gepressten Kraftträgers (1) abbilden.

10. Verfahren nach einem der Ansprüche 6 bis 9, **dadurch gekennzeichnet, dass** der Kraftträger (1) nach dem Verformungsschritt b) mindestens einen Bereich aufweist, in dem die Materialfestigkeit um mindestens 40%, vorzugsweise um mindestens 80% gegenüber der Materialfestigkeit in diesem Bereich vor der Verformung erhöht ist.

11. Verfahren nach Anspruch 10, **dadurch gekennzeichnet, dass** der Kraftträger (1) nach dem Verformungsschritt b) mindestens einen weiteren zweiten Bereich aufweist, in dem die Materialfestigkeit geringer, vorzugsweise höchstens halb so stark gegenüber dem ersten Bereich, und höher gegenüber der Materialfestigkeit in diesem Bereich vor der Verformung ist.

12. Verfahren nach Anspruch 11, **dadurch gekennzeichnet, dass** der Kraftträger (1) nach dem Verformungsschritt b) mindestens einen weiteren dritten Bereich aufweist, in dem die Materialfestigkeit geringer, vorzugsweise höchstens halb so stark, gegenüber dem zweiten Bereich und höher gegenüber der Materialfestigkeit in diesem Bereich vor der Verformung ist.

## Claims

1. Load carrier (1) for a bone fixation system, the load carrier (1) consisting of titanium, the load carrier (1) having a locally varying material strength with unchanging material elasticity, wherein the load carrier (1) has at least two adjacent holes (2, 3, 4) for holding bone screws and wherein the material of the load carrier (1) has an increased material strength in the region of the holes (2, 3, 4), **characterized in that** the holes (2, 3, 4) are provided with a means suitable for interlocking with a bone screw, in the form of a material lip (7) which does not comprise hardened material.

2. Load carrier (1) according to Claim 1, **characterized in that** the locally varying material strength corresponds to the respective load acting on these regions.

3. Load carrier (1) according to Claim 1 or 2, one of the preceding claims, **characterized in that** the load carrier (1) has two adjacent holes (2) for holding bone screws, which are to be arranged on different sides of a fracture or instability zone of a bone and the load carrier (1) has a locally increased material strength in the regions around the adjacent holes (2), or in the regions around the adjacent holes (2) and the region bridging the fracture or instability zone between the adjacent holes (2).

4. Load carrier (1) according to Claim 3, **characterized in that** the load carrier (1) has at least one further hole (3) which is arranged more distally from the fracture or instability zone of the bone compared to the first adjacent holes (2) and **in that** the load carrier (1) likewise has an increased material strength in the region around the additional hole (3) as a result of material compression, however said increase is less than, preferably only approximately half as strong as, the material strength in the region around the first adjacent holes (2).

5. Load carrier (1) according to Claim 4, **characterized in that** the load carrier (1) has at least one further hole (4), which is arranged more distally from the fracture or instability zone of the bone compared to the second hole (3), and preferably has increased material strength in the region around the further hole (4) as a result of material compression, however said increase is less than, preferably at most half as strong as, the material strength in the region around the second hole (3).

6. Method for producing a load carrier (1) according to one of Claims 1 to 5, having the following steps:
a) providing a load carrier semi-finished product (5) made of titanium, which has a greater material volume in the regions of the load carrier (1) which are intended to have a high material strength than in the regions which are intended to have a lower material strength;
b) deforming the load carrier semi-finished product (5) by means of a shaping press-form tool, so that the load carrier semi-finished product (5) is deformed between an upper tool and lower tool determining the shape of the load carrier and that the upper tool and/or lower tool have receptacles, preferably bores, which prevent significant material hardening in the regions of the load carrier semi-finished product (5) which lie opposite the receptacles during the deformation in step b) and in which holes (2, 3, 4), which are intended to be inserted, for attaching the load carrier (1) to the bone are provided;
c) holing of those regions, which lie opposite the receptacles during the deformation in step b) and in which holes for attaching the load carrier (1) to the bone are provided;
d) providing the edge of the hole which does not comprise hardened material with a material lip.

7. Method according to Claim 6, **characterized in that** the deformation by compression is effected by means of cold forming, preferably at room temperature.

8. Method according to Claim 6 or 7, **characterized in that**, in step b), the height of the load carrier semi-finished product (5) is reduced perpendicular to the deformation force in the regions which are intended to have a high material strength.

9. Method according to one of Claims 6 to 8, **characterized in that** the upper tool and/or the lower tool have inward or outward molding, preferably in the corresponding regions of the load carrier (1) which are intended to have high material strength, which image mirror-symmetrically in the surface of the pressed load carrier (1).

10. Method according to one of Claims 6 to 9, **characterized in that**, after the deformation step b), the load carrier (1) has at least one region in which the material strength is increased by at least 40%, preferably by at least 80%, compared to the material strength in this region before the deformation.

11. Method according to Claim 10, **characterized in that**, after the deformation step b), the load carrier (1) has at least one further second region, in which the material strength is less than, preferably at most half the strength of, the first region and higher than the material strength in this region before the deformation.

12. Method according to Claim 11, **characterized in that**, after the deformation step b), the load carrier (1) has at least one further third region, in which the material strength is less than, preferably at most half the strength of, the second region and higher than the material strength in this region before the deformation.

## Revendications

1. Dispositif de support de charge (1) pour un système de fixation d'os, le dispositif de support de charge (1) étant constitué de titane, le dispositif de support de charge (1) présentant localement une résistance de matériau différente pour une élasticité de matériau constante, le dispositif de support de charge (1) comprenant au moins deux trous (2, 3, 4) adjacents pour le logement de vis à os, le matériau du dispositif de support de charge (1) comprenant, au niveau des trous (2, 3, 4) une résistance de matériau augmentée, **caractérisé en ce que** les trous (2, 3, 4) sont munis de moyens adaptés à un blocage avec une vis à os, sous la forme d'une lèvre de matériau (7) non renforcée avec du matériau.

2. Dispositif de support de charge (1) selon la revendication 1, **caractérisé en ce que** la résistance de matériau localement différente correspond à la sollicitation respective de ces zones.

3. Dispositif de support de charge (1) selon la revendication 1 ou 2, **caractérisé en ce que** le dispositif de support de charge (1) comprend deux trous (2) adjacents pour le logement de vis à os, qui doivent être disposés sur des côtés différents d'une zone de fracture ou d'instabilité d'un os et le dispositif de support de charge (1) présente, au niveau des trous adjacents (2) ou au niveau des trous adjacents (2) et dans la zone reliant la zone de fracture ou la zone d'instabilité entre les trous adjacents (2), une résistance de matériau localement augmentée.

4. Dispositif de support de charge (1) selon la revendication 3, **caractérisé en ce que** le dispositif de support de charge (1) comprend au moins un trou supplémentaire (3), qui est disposé en face des premiers trous adjacents (2), de manière distale par rapport à la zone de fracture ou d'instabilité de l'os et **en ce que** le dispositif de support de charge (1) présente, au niveau du trou supplémentaire (3) également une résistance de matériau augmentée, celle-ci étant cependant plus faible, de préférence seulement la moitié de la résistance de matériau au niveau des premiers trous adjacents (2).

5. Dispositif de support de charge (1) selon la revendication 4, **caractérisé en ce que** le dispositif de support de charge (1) comprend au moins un trou supplémentaire (4) qui est disposé en face du deuxième trou (3), de manière distal par rapport à la zone de fracture ou d'instabilité de l'os et présente, de préférence au niveau du trou supplémentaire (4), une résistance de matériau augmentée, celle-ci étant cependant plus faible, de préférence seulement la moitié de la résistance de matériau au niveau du deuxième trou (3).

6. Procédé de fabrication d'un dispositif de support de charge (1) selon l'une des revendications 1 à 5, comprenant les étapes suivantes :
a) création d'un dispositif de support de charge semi-fini (5) en titane, qui présente dans les zones avec une résistance de matériau prévue importante du dispositif de support de charge (1), un volume de matériau plus important que dans les zones avec une résistance de matériau prévue plus faible ;
b) déformation du dispositif de support de charge semi-fini (5) à l'aide d'un outil de formage par compression, de façon à ce que le dispositif de support de charge semi-fini (5) soit déformé entre un outil supérieur et un outil inférieur prédéterminant la forme du dispositif de support de charge et à ce que l'outil supérieure et/ou l'outil inférieur comprennent des logements, de préférence des alésages qui empêchent une solidification importante du matériau dans les zones du dispositif de support de charge semi-fini (5) en face des logements lors de la déformation à l'étape b) et dans lesquelles les trous (2, 3, 4) à réaliser sont prévus pour la fixation du dispositif de support de charge (1) sur l'os ;
c) perforation des zones situées en face des logements lors de la déformation à l'étape b) et dans lesquelles des trous sont prévus pour la fixation du dispositif de support de charge (1) sur l'os ;
d) équipement de la paroi perforée sans solidification de matériau d'une lèvre de matériau.

7. Procédé selon la revendication 6, **caractérisé en ce que** la déformation a lieu par pressage par déformation à froid, de préférence à température ambiante.

8. Procédé selon la revendication 6 ou 7, **caractérisé en ce que**, dans l'étape b), la hauteur du dispositif de support de charge semi-fini (5) est réduite dans les zones avec une résistance de matériau prévue importante perpendiculairement à la force de formage.

9. Procédé selon l'une des revendications 6 à 8, **caractérisé en ce que** l'outil supérieur et/ou l'outil inférieur présentent des formes et des empreintes, de préférence dans les zones correspondantes du dispositif de support de charge (1) avec une résistance de matériau prévue importante, qui se forment avec une symétrie en miroir sur la surface du dispositif de support de charge (1) pressé.

10. Procédé selon l'une des revendications 6 à 9, **caractérisé en ce que** le dispositif de support de charge (1) présente, après l'étape de déformation b), au moins une zone dans laquelle la résistance du matériau est augmentée d'au moins 40%, de préférence d'au moins 80% par rapport à la résistance de matériau dans cette zone avant la déformation.

11. Procédé selon la revendication 10, **caractérisé en ce que** le dispositif de support de charge (1) présente, après l'étape de déformation b), au moins une deuxième zone dans laquelle la résistance du matériau est plus faible, de préférence au maximum la moitié de celle de la première zone, et plus élevée par rapport à la résistance de matériau dans cette zone avant la déformation.

12. Procédé selon la revendication 11, **caractérisé en ce que** le dispositif de support de charge (1) présente, après l'étape de déformation b), au moins une troisième zone dans laquelle la résistance du matériau est plus faible, de préférence au maximum la moitié de celle de la deuxième zone, et plus élevée par rapport à la résistance de matériau dans cette zone avant la déformation.
